# FASCICULE DE BREVET EUROPEEN

(11) **EP 2 117 634 B1**
(45) Date de publication et mention de la délivrance du brevet: **09.06.2010**
(21) Numéro de dépôt: 08761868.2
(22) Date de dépôt: 11.02.2008
(51) Int. Cl.: A61M 39/14, B29C 65/02, B29C 65/16, B29C 65/10, B29C 65/14

(54) **PROCEDE ET DISPOSITIF POUR RACCORDER BOUT A BOUT DES TUBES EN MATIERE PLASTIQUE**
VERFAHREN UND VORRICHTUNG ZUR VERBINDUNG DER ENDSTÜCKE VON AUS KUNSTSTOFFMATERIALIEN HERGESTELLTEN ROHREN
METHOD AND DEVICE FOR BUTT JOINTING TUBES MADE FROM PLASTIC MATERIALS

(30) Priorité: 13.02.2007 FR 0701014
(43) Date de publication de la demande: 18.11.2009
(73) Titulaire: Technord France, 59650 Villeneuve d'Ascq (FR); Optical System & Research for Industry and Science Osyris, 59260 Hellemmes (FR)
(72) Inventeur: ZEMMOURI, Jaouad, F-59242 Genech (FR); RINGOT, Jean, F-59370 Mons-En-Baroeul (FR); WASSMER, Benjamin, F-59139 Wattiginies (FR); LECOURT, Philippe, B-1430 Rebecq (BE); FOUCART, Michel, B-7540 Rumillies (BE)
(74) Mandataire: Matkowska, Franck
(86) Numéro de dépôt international: PCT/FR2008/000168
(87) Numéro de publication internationale: WO 2008/116981

(56) Documents cités:
- WO-A-02/066098
- WO-A-2005/102671

## Description

### Domaine technique

La présente invention concerne un nouveau procédé et un nouveau dispositif pour raccorder bout à bout des tubes en matière plastique. Elle trouve plus particulièrement son application dans le domaine médical à la connexion stérile de tubes en matière plastique, et par exemple, mais non exclusivement au raccordement stérile de poches de sang.

### Art antérieur

Dans le domaine médical, pour soigner un patient il est parfois nécessaire de réaliser des transfusions sanguines. Pour ce faire, on peut avoir besoin d'une grande quantité de plaquettes contenant du sang humain pour soigner le patient. D'une manière générale, la quantité de sang nécessaire pour soigner le patient est largement supérieure à la quantité de sang contenu dans une poche d'un donneur unique. Dans ce cas, il est nécessaire et courant d'extraire le sang (les plaquettes) de plusieurs poches de donneurs compatibles et de le transférer dans une unique poche de plus grande contenance.

Chaque poche de sang comporte un tube en matière thermoplastique, généralement en PVC, dont l'extrémité est fermée par écrasement du tube et soudure haute fréquence des parois du tube. Pour réaliser le transfert du sang des poches de donneurs compatibles vers une unique poche de plus grande contenance, il faut ouvrir les extrémités des tubes des poches de sang, et raccorder les extrémités ouvertes de ces tubes à un collecteur qui va diffuser le sang vers la poche de plus grande contenance. Pour éviter la contamination du sang et ne pas diminuer la durée de conservation du sang contenu dans la poche de plus grande contenance, il est important que le raccordement des extrémités des tubes soit réalisé de façon stérile. Un raccordement stérile signifie qu'aucun corps étranger n'est introduit dans les tubes pendant le raccordement, que les propriétés des matériaux utilisés pour les tubes sont inchangées (absence de brûlures), et surtout que l'air ne pénètre pas dans les tubes. Si l'ensemble de ces conditions sont réunies, l'asepsie est conservée pendant toute la durée du procédé et il ne peut y avoir de contamination du sang liée au procédé.

Par le passé, diverses solutions ont été proposées pour raccorder de façon stérile des tubes en matière plastique.

Par exemple, le document US 4 369 779 décrit un procédé et un dispositif pour raccorder de façon stérile des tubes en matière plastique, en utilisant une lame chaude coupante. Ladite lame chaude et coupante est introduite simultanément et transversalement à travers chaque tube de manière à ce que les extrémités fermées des tubes soient coupées. Les tubes sont ensuite alignés axialement par coulissement le long de la lame chaude. Puis, ladite lame chaude est rétractée et les tubes sont aboutés en vue d'être raccordés. Enfin, il est nécessaire d'exercer une traction longitudinale sur chaque tube pour ouvrir les tubes et permettre le passage d'un fluide. Le principal inconvénient de ce type de dispositif est l'utilisation d'un consommable, à savoir la lame, qui doit nécessairement être changée à chaque opération de raccordement. De plus, la stérilité n'est pas garantie pendant toute la durée du procédé. En effet, au moment de la rétractation de la lame, de l'air, voire des copeaux de plastique, peuvent pénétrer à l'intérieur des tubes et ainsi contaminer l'intérieur de ces tubes.

Le document US 4 737 214 décrit une méthode pour connecter de façon stérile des tubes en matière plastique en utilisant des moyens de chauffage des tubes par radiation et/ou convection. A cet effet, deux tubes, dont les extrémités sont soudées, sont alignés axialement et de manière à ce que lesdites extrémités soient situées à distance l'une de l'autre. Les tubes sont ensuite pincés perpendiculairement à la ligne de soudure, puis des moyens de chauffage sans contact sont déployés dans l'espace situé entre les deux extrémités de tubes. L'action combinée des moyens de chauffage et du pincement permet d'ouvrir les lignes de soudure des tubes et de rendre une forme sensiblement circulaire aux extrémités des tubes. Enfin, les moyens de chauffage sont rétractés et les extrémités ouvertes des tubes sont mises en contact pour effectuer le raccordement des deux tubes. L'avantage de cette méthode comparativement au document précité réside dans le fait qu'aucun corps étranger ne peut pénétrer à l'intérieur des tubes, car il n'y a ni coupure, ni contact, entre les extrémités des tubes et les moyens de chauffage. Toutefois, dans cet autre exemple de raccordement de tubes en matière plastique, la stérilité n'est encore pas garantie. En effet, les tubes sont ouverts avant d'être soudés et même si lesdits tubes sont pincés en amont, l'air peut pénétrer par l'ouverture produite et contaminer l'intérieur des tubes.

Le document WO 03/063940 décrit un laser pour la soudure de tubes à usage médical. Le laser est notamment utilisé pour raccorder des tubes en matière plastique. Les tubes sont initialement écartés l'un de l'autre, et leurs extrémités sont soumises à l'action du laser pour être chauffées. Une fois chauffées, les extrémités sont rapprochées et raccordées bout à bout. Un inconvénient du système décrit dans ce document est que le faisceau laser doit être divisé en deux faisceaux secondaires qui sont déviés en direction des deux extrémités écartées des deux tubes. De plus, comme dans les documents précités, la stérilité n'est pas garantie car les extrémités des tubes sont ouvertes avant d'être aboutées, et de l'air peut alors pénétrer à l'intérieur des tubes et les contaminer.

Le document WO 2005/102671 décrit une méthode pour raccorder de façon stérile des tubes en utilisant un laser. Le laser permet selon la méthode décrite de souder ensemble les extrémités de deux tubes en aboutant lesdites extrémités sur un film absorbant. A cet effet, deux extrémités de tubes sont disposées en vis-à-vis l'une de l'autre, leur section s'étendant sensiblement parallèlement, puis on vient placer un film absorbant l'énergie du laser entre les deux extrémités, les extrémités des tubes sont alors au contact du film. Enfin, on dirige le laser vers le film et les extrémités à raccorder. L'inconvénient principal de cette méthode est que le film absorbant participe au soudage des deux extrémités. En effet, un résidu de film, qui est déchiré au moment de l'ouverture des tubes, se trouve emprisonné à l'intérieur des tubes. Cette méthode est compliquée et délicate à mettre en oeuvre, et le contact entre le film et les tubes préalablement au soudage, ainsi que le résidu de film emprisonné à l'intérieur du tube, compromettent la stérilité des tubes.

### Objectif de l'invention

La présente invention vise principalement à proposer une nouvelle solution technique pour raccorder bout à bout deux tubes en matière plastique dont les extrémités sont initialement fermées, sans que de l'air et/ou des corps étrangers ne puissent pénétrer à l'intérieur des tubes.

### Résumé de l'invention

L'invention a ainsi pour premier objet un procédé de raccordement bout à bout de deux tubes en matière plastique dont les extrémités sont fermées par écrasement et soudure ou collage de la paroi du tube.

Selon ce procédé, on aligne axialement et on aboute les deux extrémités fermées des tubes, on chauffe à distance et sans contact les deux extrémités fermées et aboutées des tubes et on rapproche axialement les deux extrémités des tubes, en sorte d'évaser et de souder ensemble bout à bout les extrémités des tubes, tout en maintenant lesdites extrémités constamment aboutées.

De préférence, mais de manière facultative selon l'invention, le procédé selon l'invention comporte les caractéristiques techniques additionnelles ci-après, prises isolément ou en combinaison :
- pour chauffer à distance et sans contact les deux extrémités fermées et aboutées des tubes, on utilise un faisceau laser et l'un au moins des moyens choisis parmi la liste suivante : flux d'air chaud, rayonnement micro-ondes, rayonnement infrarouge ;
- de préférence, en cas d'utilisation d'un faisceau laser celui-ci est orienté transversalement au plan d'écrasement des deux extrémités, et touche simultanément les deux extrémités fermées et aboutées des tubes ; le cas échéant, le faisceau laser effectue un balayage des extrémités fermées et aboutées des tubes suivant une direction (T) transversale à l'axe des tubes ;
- dans une variante de réalisation, on chauffe à distance et sans contact les deux extrémités fermées et aboutées des tubes à l'intérieur d'une enceinte ;
- dans une variante de réalisation, on détecte la température des extrémités des tubes, et on commande le rapprochement des tubes en fonction de cette détection de température ;
- postérieurement au raccordement des deux tubes on exerce une traction sur les tubes raccordés ;
- de préférence, mais non nécessairement, les extrémités sont initialement fermées par soudure haute fréquence et/ou les tubes sont en PVC.

L'invention a pour autre objet un dispositif de raccordement de tubes, permettant la mise en oeuvre du procédé visé précédemment et comprenant des moyens d'aboutement permettant un alignement axial et un aboutement de deux tubes, et des moyens de chauffage sans contact; les moyens d'aboutement sont conçus pour déplacer automatiquement en translation l'une vers l'autre les deux extrémités aboutées des deux tubes pendant la mise en oeuvre des moyens de chauffage.

De préférence, mais de manière facultative selon l'invention, le dispositif selon l'invention comporte les caractéristiques techniques additionnelles ci-après, prises isolément ou en combinaison :
- le dispositif comprend des moyens optiques de détection de température positionnés à proximité de la zone d'aboutement des tubes, et les moyens d'aboutement sont aptes à entrainer en translation les tubes en fonction du signal de détection de température délivré par lesdits moyens de détection ;
- le dispositif comprend une enceinte de chauffage et les moyens d'aboutement comportent des moyens d'entrainement en translation des tubes, qui sont à l'intérieur de l'enceinte ;
- les moyens d'aboutement comportent deux chariots alignés qui sont mobiles et guidés en translation, chaque chariot étant équipé d'un moyen permettant de fixer temporairement un tube sur le chariot, et des moyens d'actionnement permettant de rapprocher ou d'écarter les chariots ;
- dans une première variante, les moyens d'actionnement des chariots comportent une vis sans fin et un moteur bidirectionnel couplé à la vis et permettant d'entraîner la vis en rotation dans un sens ou dans l'autre ;
- dans une deuxième variante les moyens d'actionnement des chariots comportent pour chaque chariot au moins un actionneur linéaire bidirectionnel, de type vérin.
- dans une troisième variante, les moyens d'actionnement comportent des moyens élastiques exerçant constamment sur les chariots des forces de rappel les repoussant l'un vers l'autre ;
- De préférence, les moyens de chauffage sans contact comportent une source laser et/ou au moins une buse de soufflage d'air chaud et/ou une source micro-ondes et/ou une source infrarouge.

L'invention a pour autre objet l'utilisation du procédé ou du dispositif visé précédemment pour raccorder de manière stérile les tubes fermés de deux poches, dont l'une contient des plaquettes de sang ou un autre fluide nécessitant les mêmes exigences d'asepsies.

### Brève description des figures

L'invention et ses avantages seront mieux compris à la lecture de la description qui va suivre, donnée uniquement à titre d'exemple non limitatif et non exhaustif, et faite en se référant aux figures annexées parmi lesquelles :
- La figure 1 représente schématiquement le raccordement de poches de sang à un collecteur dans le domaine médical de la transfusion sanguine,
- La figure 2 est une représentation en perspective d'un exemple de tube en matière plastique, dont l'extrémité est fermée de manière étanche, par écrasement et soudure (ou collage) de la paroi du tube,
- la figure 3 est une représentation en perspective d'une première variante de réalisation d'un dispositif selon l'invention,
- la figure 4 est une vue de dessus du dispositif de la figure 3, les moyens de chauffage par air chaud n'étant pas représentés,
- la figure 5 est une vue de face du dispositif de la figure 3, les moyens de chauffage par air chaud n'étant pas représentés,
- la figure 6 est un schéma bloc des moyens électroniques permettant une commande automatique du fonctionnement du dispositif de raccordement (commande du laser et des moyens de chauffage par air chaud et commande du déplacement des chariots)
- les figures 7 et 8 représentent deux tubes montés sur le dispositif de la figure 3 (seuls les chariots et les moyens de clam page sont représentés) dans une position intermédiaire du procédé (en phase de rapprochement des chariots),
- les figures 9 et 10 représentent deux tubes montés sur le dispositif de la figure 3 (seuls les chariots et les moyens de clampage sont représentés) dans une position finale du procédé,
- les figues 11 et 12 représentent deux tubes alignés axialement et aboutés,
- les figures 13 et 14 représentent deux tubes aboutés dans la phase de rapprochement des chariots du dispositif de la figure 3,
- les figures 15 et 16 représentent deux tubes raccordés bout à bout, à l'issue du procédé de raccordement de l'invention,
- la figure 17A est une représentation en perspective d'une deuxième variante de réalisation d'un dispositif selon l'invention,
- la figure 17B est une représentation en perspective d'une troisième variante de réalisation d'un dispositif selon l'invention,
- les figures 18 à 23 illustrent des étapes additionnelles et facultatives pour la réalisation de soudures d'extrémité secondaires, parfaitement calibrées et contrôlées, en remplacement des soudures d'extrémité initiales.

### Description détaillée

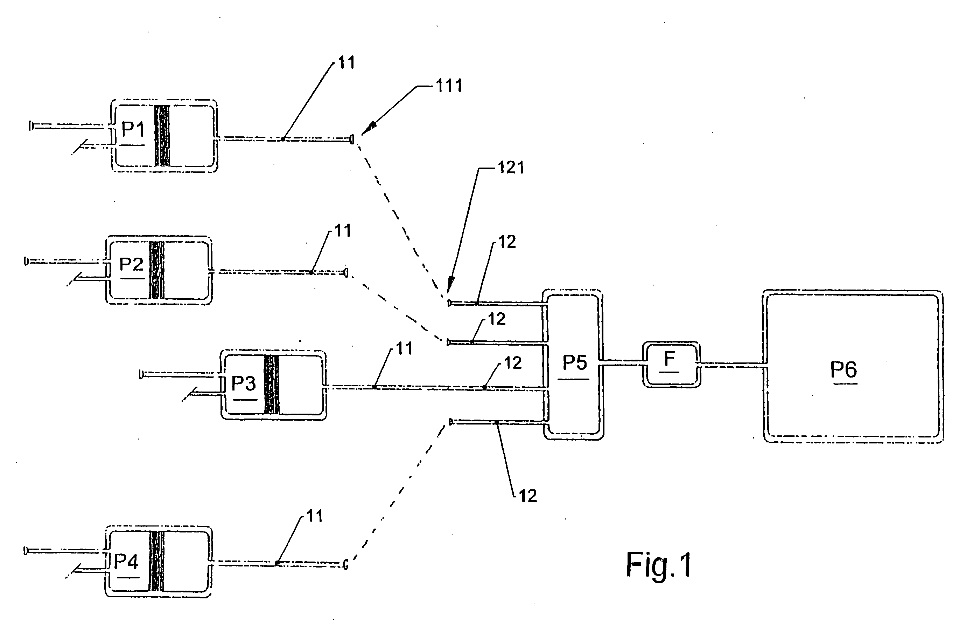

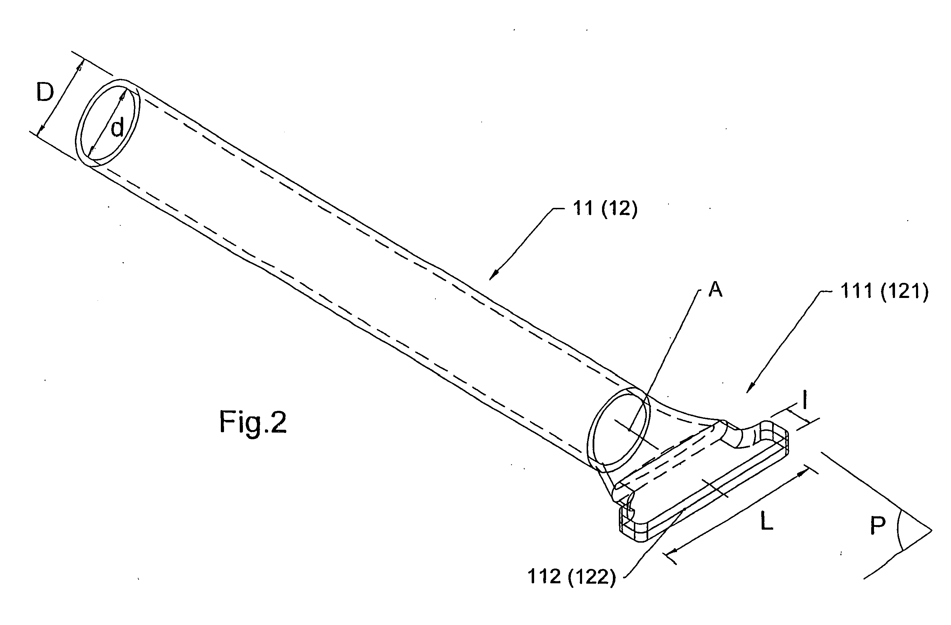

### Exemple d'application - Raccordement stérile de poches contenant des plaquettes de sang (Figures 1 et 2)

Une des applications préférentielles, mais non exclusive et non limitative de l'invention, se situe dans le domaine médical de la transfusion sanguine, et consiste dans le raccordement stérile de poches contenant des concentrés de plaquettes de sang. Les niveaux et les exigences en matière de stérilité varie selon les applications (médicales, agro-alimentaires ...), cependant, c'est dans le domaine médical de la transfusion sanguine que ces niveaux et exigences sont les plus élevés. Ainsi, l'asepsie doit impérativement être préservée.

Pour soigner un patient il est parfois nécessaire de réaliser des transfusions sanguines. Pour ce faire, on peut avoir besoin d'une grande quantité de plaquettes de sang humain pour soigner le patient. D'une manière générale, la quantité de plaquettes nécessaire pour soigner le patient est largement supérieure à la quantité de plaquettes contenue dans une poche d'un donneur unique. Dans ce cas, il est nécessaire et courant d'extraire les plaquettes de plusieurs poches de donneurs compatibles et de les transférer dans une unique poche de plus grande contenance.

A titre d'exemple, on a représenté sur la figure 1 quatre poches intermédiaires P1, P2, P3 et P4 contenant chacune un concentré de plaquettes, une poche P5 communément désignée collecteur, et une poche finale P6 de plus grande contenance que les poches intermédiaires P1 à P4. Le collecteur P5 est raccordé à la poche finale P6, un filtre F étant de préférence interposé entre le collecteur P5 et la poche finale P6.

Chaque poche intermédiaire P1 à P4 comporte un tube souple 11 en matière thermoplastique, dont l'extrémité 111 est fermée de manière étanche par écrasement et soudure haute fréquence de la paroi du tube 11. Le collecteur P5 comporte quatre tubes souples 12 permettant son remplissage. L'extrémité 121 de chaque tube 12 est fermée de manière étanche par écrasement et soudure haute fréquence de la paroi du tube 12.

Les extrémités 111,121 écrasées et soudées des tubes 11, 12 apparaissent de manière plus détaillée sur la figure 2. Sur cette figure 2, et dans la suite de la description, le plan d'écrasement des extrémités 111,121 est référencé (P), et les soudures d'extrémité des tubes 11 et 12 sont référencées respectivement 112 et 122. Ces zones 112, 122 aplaties et soudées à l'extrémité des tubes 11,12 s'étendent sur une largeur (I) selon la direction axiale (A) du tube, sur une longueur (L), et sur une très faible épaisseur, qui est suffisante pour fermer hermétiquement l'extrémité du tube.

Pour réaliser le transfert des concentrés de plaquettes des poches intermédiaires P1 à P4 vers la poche P6 de plus grande contenance, il faut raccorder les poches intermédiaires P1 à P4 au collecteur P5. Pour raccorder une poche intermédiaire P1, P2, P3 ou P4 au collecteur P5, il faut ouvrir l'extrémité fermée 111 du tube 11 de cette poche intermédiaire, ainsi que l'extrémité fermée 121 de l'un des tubes 12 du collecteur P5, et raccorder bout à bout les extrémités ouvertes de ces deux tubes 11 et 12. Sur la figure 1, le tube 11 de la poche P3 est raccordé bout a bout à l'un des tubes 12 du collecteur P5.

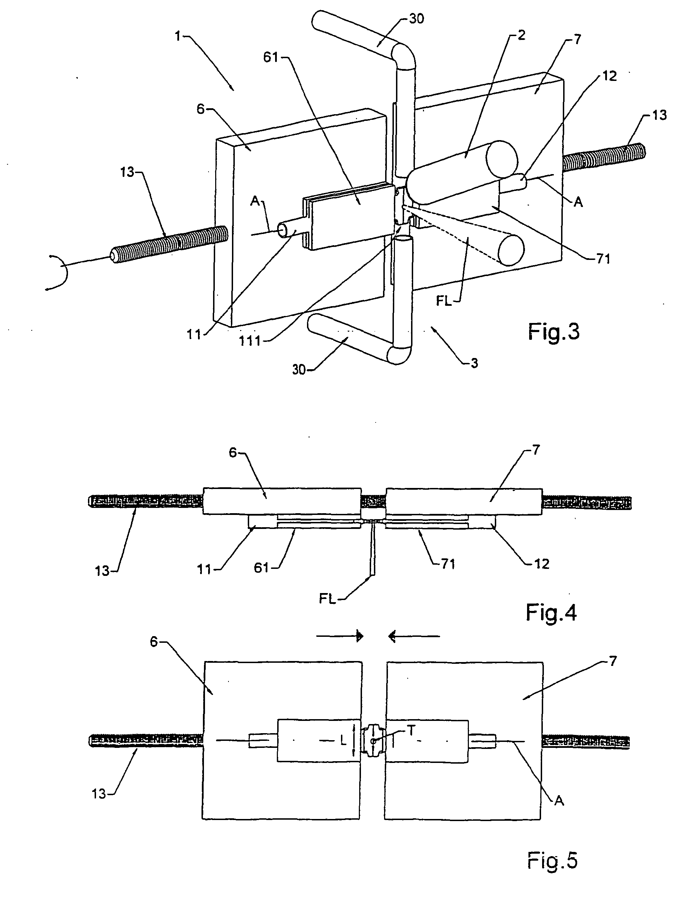

Pour éviter la contamination des plaquettes de sang transférées dans la poche de plus grande contenance P6, et ne pas diminuer leur durée de conservation, il est important que le raccordement des extrémités des tubes 11,12 soit réalisé de façon stérile. Un raccordement stérile signifie qu'aucun corps étranger n'est introduit dans les tubes 11, 12 pendant leur raccordement, que les propriétés du matériau des tubes 11,12 sont inchangées (absence de brûlures), et surtout que l'air ne pénètre pas dans les tubes 11,12. Si ces conditions sont réunies, l'asepsie est conservée pendant toute la durée du procédé et il ne peut y avoir de contamination des plaquettes de sang. Dispositif de raccordement de tubes - Structure- Première variante (figures 3 à 6)

On a représenté sur les figures 3 à 6, une première variante de réalisation d'un dispositif 1 de l'invention qui est destiné à être utilisé pour effectuer le raccordement bout à bout de tubes fermés 11,12. Ce dispositif 1, dont la structure et le fonctionnement vont à présent être détaillés, permet avantageusement d'effectuer cette opération de raccordement de tubes 11,12, de façon stérile, avec un temps de cycle court, et sans nécessiter l'utilisation d'outils consommables, en particulier pour l'ouverture des tubes 11,12.

Ce dispositif 1 est avantageusement utilisé dans le domaine médical de la transfusion sanguine pour le raccordement de tubes 11,12 de poches lors d'une opération précitée de transfert de plaquettes de sang.

Plus généralement, le dispositif de l'invention peut être utilisé dans toute application où il est nécessaire de raccorder bout à bout deux tubes en matière plastique, et plus particulièrement en matière thermoplastique, dont les extrémités respectives 111 et 121 sont initialement fermées par écrasement et soudure ou collage de la paroi du tube. L'invention n'est pas limitée à des tubes fermés par soudure HF, mais peut être appliquée à tout tube plastique comportant une extrémité fermée par écrasement et soudure ou collage de la paroi du tube, et pouvant être ouverte par chauffage..

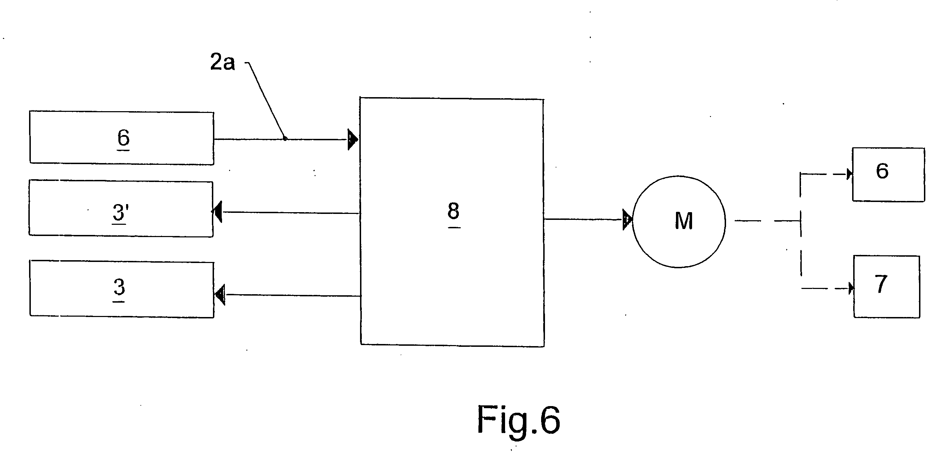

En référence aux figures 3 à 6, le dispositif 1 de raccordement de tubes comprend deux chariots mobiles 6 et 7, des moyens de chauffage par flux d'air chaud 3, une source laser 3' [figure 6] apte à délivrer un faisceau laser FL, un pyromètre 2, et des moyens d'actionnement des chariots 6,7 comportant notamment des moyens électronique de commande 8 [figure 6]. Sur les figures 3 et 4, la source laser n'est pas représentée, et seul le faisceau laser FL produit au moyen de cette source laser a été représenté. Le faisceau laser FL est destiné à être appliqué sur les soudures d'extrémité 112, 122 des deux tubes 11 et 12, afin de chauffer lesdites soudures, et permettre notamment l'ouverture des extrémités 111, 121 des tubes. Ce faisceau laser FL est orienté transversalement au plan d'écrasement (P) des deux extrémités 111, 121 et touche simultanément les deux extrémités fermées et aboutées 111, 121 des tubes 11,12. La longueur d'onde d'émission du laser est choisie en fonction du type de matériau constituant les tubes 11, 12, de manière à ce que l'énergie délivrée par le faisceau laser FL soit absorbée de manière efficace par les parois des tubes 11,12. La puissance du laser sera choisie notamment en fonction de la rapidité d'action souhaitée pour le laser.

Le diamètre du faisceau laser FL est de préférence inférieur ou égal à la largeur (I) des soudures (par exemple 1mm), mais est généralement inférieur à la longueur (L) des soudures 112, 122. Dans ce cas, il est préférable que le faisceau laser FL balaye les soudures 112, 122 des tubes 11, 12 suivant leur longueur (L), c'est-à-dire en référence à la figure 5 dans la direction T transversale à l'axe A des tubes 11, 12. Ce balayage est réalisé sur tout ou partie de cette longueur L. Le laser équipant le dispositif 1 présente par exemple une fréquence de balayage des soudures 112, 122 de l'ordre de 0,5 Hz, c'est-à-dire que le faisceau laser FL réalise un aller-retour sur les soudures 112, 122 en deux secondes.

Les deux chariots 6 et 7 sont montés sur une vis sans fin 13, et sont mobiles en translation le long de cette vis. Cette vis sans fin 13 est entraînée en rotation sur elle-même par un moteur. La rotation de la vis sans fin 13 dans un sens provoque le rapprochement des deux chariots 6,7 et la rotation de la vis sans fin 13 dans l'autre sens provoque l'écartement des deux chariots 6,7 à une vitesse dépendant notamment de la vitesse de rotation de la vis 13. Les chariots 6, 7 sont équipés de moyens de clampage 61, 71 qui permettent de fixer temporairement sur les chariots les extrémités des tubes 11, 12, et d'évacuer le cas échéant du liquide contenu à l'intérieur des extrémités 111, 121 des tubes 11, 12, avant les opérations de raccordement. Les deux chariots 6,7 et leurs moyens de clampage 61,71 sont alignés en sorte de permettre l'alignement axial des tubes 11 et 12 devant être raccordés.

Les moyens de chauffage par flux d'air chaud 3 comportent deux buses de soufflage 30, qui sont alimentées en air chaud. Ces buses 30 débouchent à proximité de la zone de raccordement des tubes 11,12. Ce flux d'air chaud permet d'accélérer le chauffage des extrémités 111, 121 des tubes 11 et 12, et ainsi de réduire le temps de cycle de raccordement des tubes.

Le pyromètre 2 est un capteur permettant de détecter sans contact et de manière optique (généralement par détection infrarouge) la température des tubes. De préférence, le pyromètre 2 utilisé dans le dispositif 1 est compact, et est monté à proximité de la zone de raccordement des tubes 11,12. La température détectée par ce pyromètre est une température moyenne prise dans le champ de vision du pyromètre, les parois des extrémités 111,121 des tubes 11,12 étant comprises dans le champ de vision.

En référence à la figure 6, les moyens électroniques de commande 8 du dispositif 1, reçoivent en entrée le signal de détection de température 2a délivré par le pyromètre 2, et permettent de commander les moyens de chauffage (source laser 3' et alimentation en air chaud des buses de soufflage 30 des moyens de chauffage par flux d'air chaud 3, ainsi que le moteur (M) permettant la mise en rotation de la vis sans fin 13 dans un sens ou dans l'autre (pour le déplacement en translation des chariots 6,7). Ces moyens électroniques de commande 8 comportent une unité de commande programmable (implémentée par exemple au moyen d'un microprocesseur), qui est conçue pour exécuter automatiquement un programme de commande stocké en mémoire. L'exécution automatique de ce programme de commande permet aux moyens de commande 8 de piloter les moyens de chauffage (laser et alimentation en air chaud des buses 30) et le déplacement des chariots 6,7 en fonction de la température détectée par le pyromètre 2. Un exemple de programme de commande est détaillé ci-après.

### Mise en oeuvre et fonctionnement du dispositif de raccordement

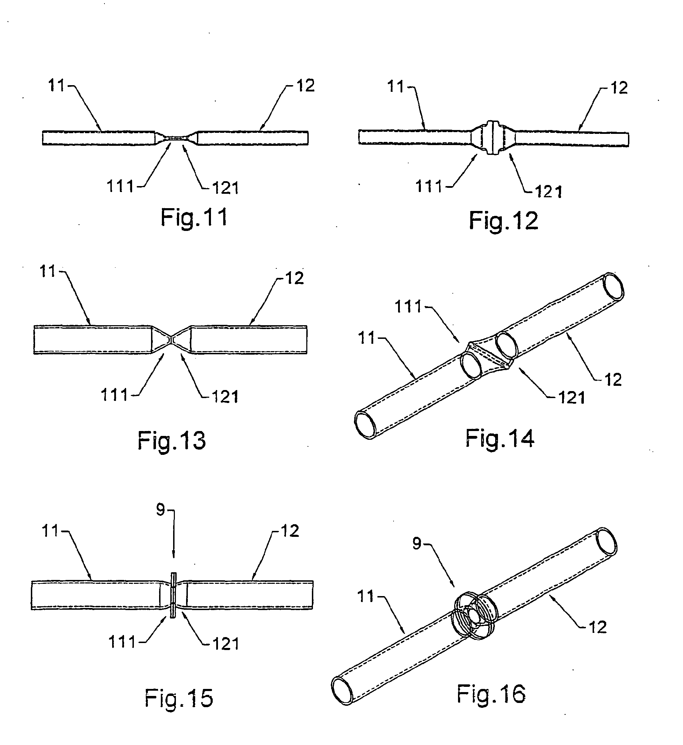

Un exemple particulier de mise en oeuvre et de fonctionnement du dispositif de raccordement 1 précité pour connecter bout à bout deux tubes 11,12 fermés va à présent être détaillé en référence aux figures 3 à 16. Les figures 11 à 16 représentent les principales étapes du procédé de raccordement, mais seuls les tubes 11 et 12 sont représentés pour une améliorer la clarté et la compréhension du procédé.

Pour connecter bout à bout deux tubes 11,12 fermés, au moyen du dispositif 1, on procède de la manière suivante.

### 1^{ère} étape

On positionne chaque tube 11, 12 sur un chariot 6, 7 et on le fixe temporairement sur le chariot à l'aide des moyens de clampage 61, 71. Tel que cela est représenté les figures 3 à 5, et sur les figures 11 et 12, les deux tubes 11 et 12 sont alignés axialement et sont aboutés, leurs extrémités fermées 111 et 121 étant au contact l'une de l'autre. Pour accélérer le procédé, on peut préalablement à cette étape ou au cours de cette étape mettre en route les moyens de chauffage par flux d'air chaud.

### 2^{ème} étape

On met en route le laser et les moyens de chauffage par flux d'air chaud 3 (alimentation en air chaud des buses de soufflage 30), ce qui permet de démarrer un chauffage sans contact des extrémités 111, 121 fermées et aboutées des deux tubes 11,12, avec !e cas échéant un balayage par le laser des soudures d'extrémité 112,122 des deux tubes 11,12.

Cette première étape de chauffage est poursuivie jusqu'à ce que la température mesurée par le pyromètre 2 atteigne un premier seuil de température (T1) prédéfini.

### 3^{ème} étape

Lorsque la température mesurée par le pyromètre 2 atteint le premier seuil de température T1, les moyens de commande 8 pilotent le moteur (M) d'entraînement des vis sans fin 13, en sorte de rapprocher les deux chariots 6,7 l'un vers l'autre à une vitesse V1 prédéfinie.

Cette étape de chauffage est poursuivie jusqu'à ce que la température mesurée par le pyromètre 2 atteigne un deuxième seuil de température T2 prédéfini (T2 > T1).

### 4^{ème} étape

Lorsque la température mesurée par le pyromètre 2 atteint le deuxième seuil de température T2, les moyens de commande 8 pilotent le moteur (M) d'entraînement de la vis sans fin 13, en sorte de rapprocher les deux chariots 6,7 l'un vers l'autre à une vitesse V2 prédéfinie supérieure à la vitesse V1 précitée.

Cette étape de chauffage est poursuivie jusqu'à ce que la température mesurée par le pyromètre 2 atteigne un troisième seuil de température T3 prédéfinie (T3 > T2).

### 5^{ème} étape

Lorsque la température mesurée par le pyromètre 2 atteint le troisième seuil de température T3, les moyens de commande 8 arrêtent le moteur (M) (immobilisation des chariots 6 et 7), arrêtent le laser et coupent l'alimentation en air chaud des buses de soufflage 30.

Au cours des étapes de chauffage précitées, les extrémités 111, 121 fermées et aboutées des deux tubes 11,12 sont chauffées jusqu'à atteindre la température de transition vitreuse du matériau, soit par exemple entre 130°C et 150°C pour !e PVC. La transition vitreuse correspond à une augmentation de la viscosité du tube qui permet malgré tout d'assurer un maintien de la forme du tube.

Le chauffage localisé par laser et flux d'air chaud des extrémités 111, 121 aboutées des deux tubes 11, 12 altère les deux soudures d'extrémité 112, 122 et provoque le ramollissement du matériau au niveau des extrémités des tubes 11,12.

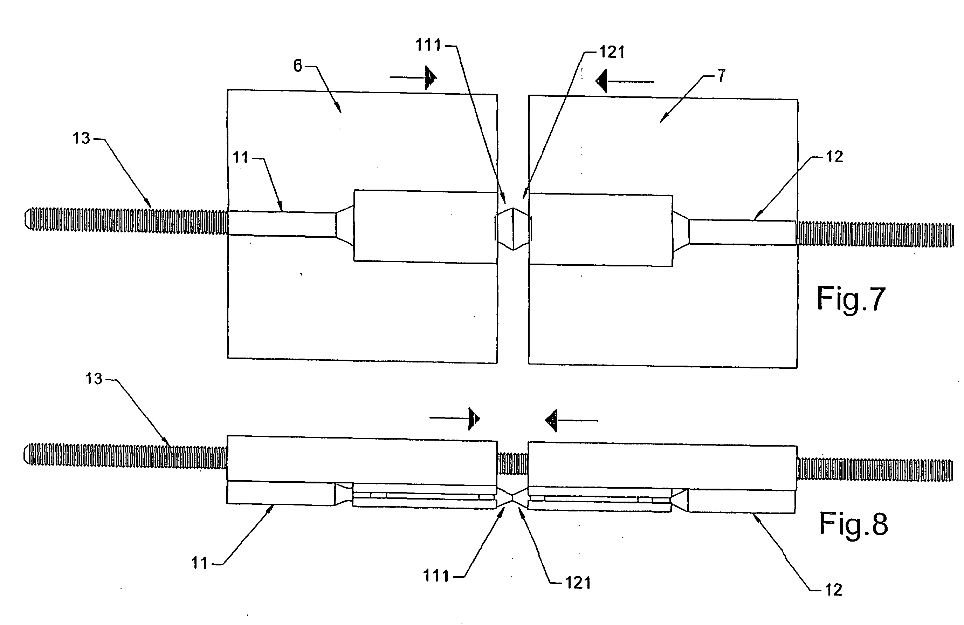

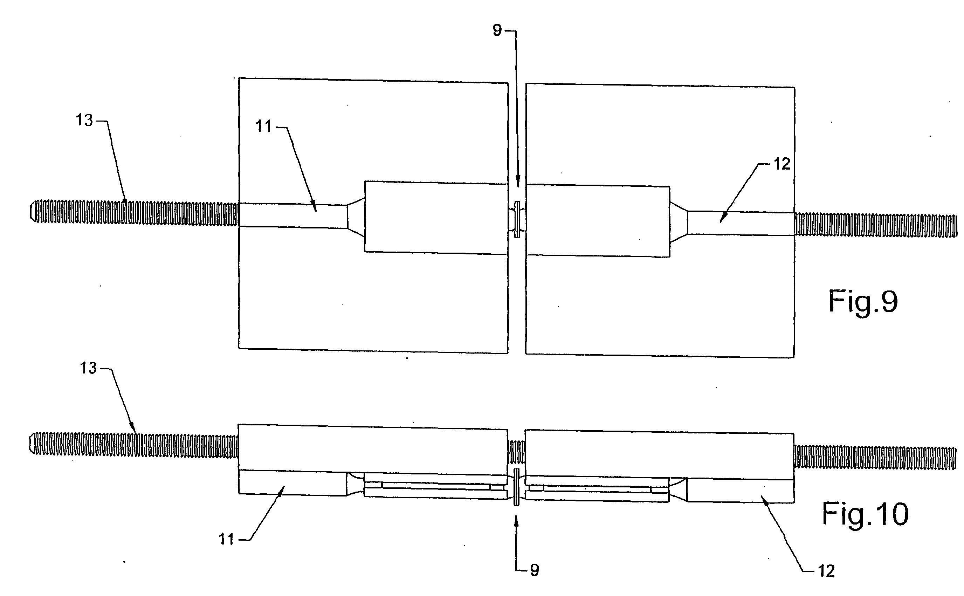

Le rapprochement des chariots 6 et 7 au cours des 3^{ène} et 4^{ème} étapes précitées permet dans un premier temps de compenser le retrait de matière découlant du ramollissement du matériau et de maintenir les tubes 11,12 constamment aboutés (figures 7, 8 et figures 13, 14) ; dans un second temps, le rapprochement des chariots 6 et 7 permet de rapprocher suffisamment l'un de l'autre les deux tubes 11,12 en exerçant une pression qui permet la formation d'une connexion 9 entre les tubes 11 et 12 (figures 9, 10 et figures 15, 16). La pression exercée lors de ce rapprochement des deux tubes 11,12 permet d'évaser les deux extrémités aboutées 111, 121 des deux tubes 11, 12, et participe ainsi à l'ouverture des tubes 11,12 (relachement des soudures d'extrémité).

Généralement, à l'issue de la 5^{ème} étape précitée, les deux tubes 11, 12 raccordés bout à bout communiquent entre eux et permettent le passage d'un fluide à l'intérieur des tubes 11,12. Toutefois, dans certain cas, du fait de l'écrasement initial des extrémités 111,121 des tubes 11 et 12, il peut arriver qu'un fin film se crée au niveau de la connexion 9 lors de l'ouverture des tubes 11,12 et bouche ainsi lesdits tubes 11,12. Pour remédier à ce problème, on peut inclure dans le procédé une étape supplémentaire et postérieure à la soudure des deux tubes dans laquelle les moyens d'aboutement agissent, par l'intermédiaire des moyens d'actionnement sur les chariots 6, 7 en sorte d'écarter légèrement les chariots 6,7. Cet écartement a pour effet d'exercer une traction sur les deux tubes 11,12 raccordés bout à bout, ladite traction étant suffisante pour déchirer ledit film et libérer le passage pour un fluide, sans déconnecter les deux tubes.

A l'issue du procédé, les deux tubes 11 et 12 sont raccordés bout à bout tel que cela est illustré sur les figures 15 et 16, et communiquent entre eux, ce qui permet le passage d'un fluide d'un tube à l'autre.

Les seuils de température T1, T2, T3 et les vitesses de déplacement des chariots V1 et V2 sont déterminés au cas par cas par l'homme du métier, en sorte d'obtenir le raccordement bout à bout des tubes 11,12 en maintenant les deux extrémités 111, 121 de tubes 11,12 constamment aboutées. Ce maintien de l'aboutement est important car il permet d'éviter une contamination de l'intérieur des tubes 11, notamment avec de l'air provenant de l'extérieur des poches, et contribue à préserver la stérilité et l'asepsie des tubes 11 et 12.

A titre d'exemple non limitatif de l'invention, dans un exemple précis de réalisation, le dispositif de raccordement 1 a été utilisé pour connecter bout à bout des tubes 11, 12 identiques à celui représenté sur la figure 2. Les tubes 11 et 12 étaient en PVC ; Le diamètre externe (D) des tubes 11, 12 valait environ 4 mm et le diamètre interne (d) des tubes valait environ 3 mm. Les soudures HF 112, 122 à l'extrémité des tubes 11,12 étaient réalisées dans une zone qui s'étendait sur une largeur (I) de 1 mm selon la direction axiale du tube, sur une longueur (L) de 8 mm, et sur une épaisseur de l'ordre de 0,5 mm.

Le laser équipant le dispositif 1 était conçu pour émettre dans la gamme de longueur d'ondes entre 1,5 et 2,1 µm, et avec une puissance de 2W à 3W. L'air chaud soufflé par les buses 30 était porté à une température comprise entre 400°C et 650°C.

Le pyromètre 2 utilisé était le capteur sans contact infrarouge commercialisé par la société CALEX sous la référence EL21 et était apte à détecter des températures supérieures à 15°C avec une précision de +/-1°C, et avec un temps de réponse rapide, de l'ordre de 200 ms. Le pyromètre 2 était situé à environ 5 cm de la zone de raccordement des tubes 11, 12, entre les deux chariots 6,7, et le diamètre de la zone de mesure de température était de l'ordre de 35,5 mm.

Les seuils de température T1, T2 et T3 valaient respectivement 31°C, 40°C et 43°C. Les vitesses de consigne V1 et V2 valaient respectivement 150µm/s et 200µm/s.

La mise en oeuvre d'un chauffage par laser permet avantageusement d'obtenir un chauffage localisé des soudures d'extrémité 112, 122 des tubes, agissant directement au coeur du matériau, ce qui permet d'obtenir une destruction par chauffage des soudures 112, 122, plus efficace et plus rapide. L'apport d'un flux d'air chaud en combinaison avec le laser permet d'accélérer le procédé, notamment pour obtenir le soudage bout à bout des tubes.

L'invention n'est toutefois pas limitée à la mise en oeuvre d'un laser combiné à un flux d'air chaud. Il est envisageable d'utiliser par exemple uniquement un laser ou uniquement de l'air chaud. Plus généralement, on peut utiliser tout moyen de chauffage connu (par rayonnement, notamment rayonnement micro-onde ou infrarouge et/ou convection), permettant de chauffer à distance et sans contact les extrémités des tubes.

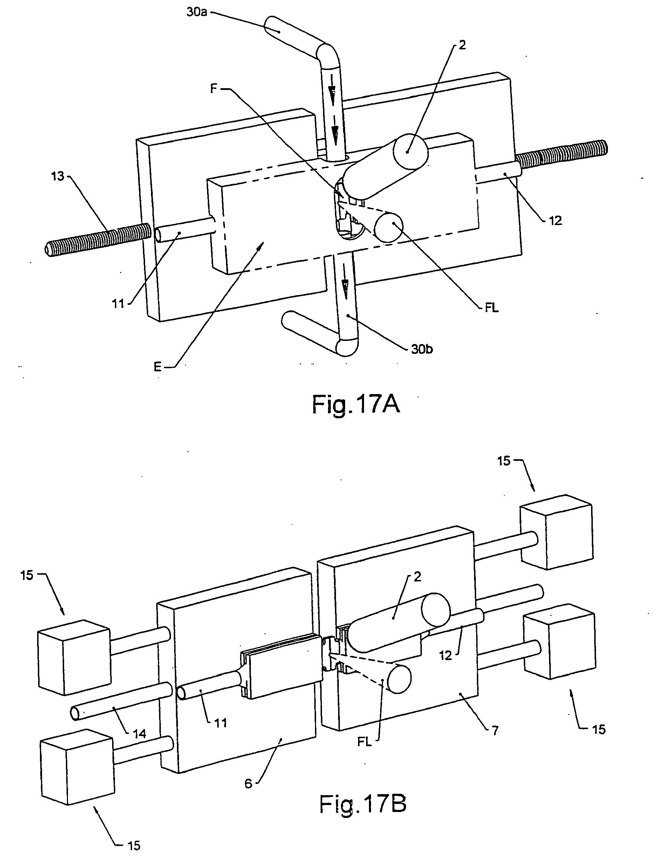

On a représenté sur la figure 17A, une autre variante de réalisation d'un dispositif de l'invention, qui se différencie de la variante précitée des figures 3 à 5, par la mise en oeuvre d'une enceinte E et de moyens de chauffage 30a, 30b du volume intérieur de l'enceinte. Les moyens d'entraînement (chariots 6,7) des tubes sont logés à l'intérieur de cette enceinte E. Cette enceinte E est de préférence mais non nécessairement fermée de manière étanche, à l'exception des deux orifices en vis-à-vis permettant le passage des tubes 11, 12. Les moyens de chauffage de l'enceinte comportent une buse de soufflage 30a alimentée en air chaud et une buse d'aspiration 30b. Ces buses 30a et 30b débouchent en vis-à-vis l'une de l'autre à l'intérieur de l'enceinte E, et à proximité de la zone d'aboutement des extrémités 111,121 des tubes 11,12. La buse de soufflage 30a permet, lorsqu'elle est alimentée en air chaud, d'introduire de l'air chaud à l'intérieur de l'enceinte E, et la buse d'aspiration 30b permet de recycler l'air contenu à l'intérieur de l'enceinte E vers l'extérieur de l'enceinte E. En fonctionnement, ces buses 30a, 30b permettent de créer un flux d'air chaud dans la zone d'aboutement des tubes à l'intérieur de l'enceinte E, et d'élever la température de l'air à intérieur de l'enceinte E. L'enceinte E comporte en outre une fenêtre F, transparente pour le faisceau laser FL (par exemple fenêtre en en quartz).

De manière avantageuse selon cette variante, on peut préchauffer le volume intérieur de l'enceinte par l'apport d'un flux d'air chaud au moyens des buses 30a, 30b, et avant d'introduire dans l'enceinte E les deux tubes 11,12 à raccorder. Ce préchauffage permet non seulement d'éliminer toutes les bactéries pouvant se trouver à l'intérieur de l'enceinte E, mais aussi de réduire la durée d'un cycle de raccordement de deux tubes 11,12.

Dans les variantes de réalisation qui viennent d'être décrites, l'actionnement des chariots 6,7 est commandé en fonction d'une mesure de température au moyen d'un pyromètre 2. D'une part, on peut remplacer ce pyromètre 2, par tout autre moyen connu de mesure de température équivalent, et par exemple par une caméra thermique reliée aux moyens de commande 8.

Au surplus, la mise en oeuvre d'un contrôle de température au moyen d'un capteur thermique 2 est préférable mais n'est toutefois pas indispensable. Dans une autre variante de réalisation, les moyens de commande 8 peuvent par exemple être programmés pour commander le déplacement des chariots avec des temps de cycle prédéfinis. Par exemple, les durées des 2^{ème}, 3^{ème} et 4^{ème} étapes peuvent être prédéfinies. Dans ce cas l'utilisation d'un capteur de température 2 n'est pas nécessaire.

Les moyens d'aboutement des deux tubes ne comportent pas nécessairement deux chariots 6,7 mobiles en translation, lesdits chariots 6,7 pouvant être remplacés par tout moyen équivalent permettant d'abouter deux tubes, et de déplacer en translation les deux tubes l'un vers l'autre, en maintenant les deux tubes aboutés.

Les moyens de déplacement en translation des chariots (vis sans fin 13, moteur M) peuvent être remplacés par tout autre moyen permettant de rapprocher ou d'écarter les deux chariots 6,7.

En particulier, le déplacement des chariots 6, 7 peut être piloté par les moyens de commande 8 au moyen de tout actionneur linéaire bidirectionnel. Par exemple en référence à la figure 17B, dans une autre variante de réalisation les deux chariots 6, 7 sont montés et coulissent en translation sur une tige de guidage fixe 14, et les deux chariots 6,7 peuvent être déplacés le long de cette tige 14 au moyen de vérins électriques 15 bidirectionnels. Ces vérins 15 sont pilotés par les moyens électroniques de commande 8

Dans une autre variante de réalisation plus simple, le dispositif ne comporte pas nécessairement de moyens de commande électroniques 8, et le déplacement des chariots 6, 7 est commandé de manière purement mécanique. Par exemple, les deux chariots 6,7 peuvent être guidés en translation en étant montés sur une tige de fixe ou équivalent, et les moyens d'actionnement des chariots comportent des moyens élastiques (par exemple des ressorts de rappel) exerçant constamment sur les chariots 6,7 des forces de rappel les repoussant l'un vers l'autre. Dans ce cas, lors du ramollissement du matériau découlant du chauffage des extrémités aboutées 111, 121 de deux tubes 11,12, les ressorts repoussent mécaniquement les deux tubes 11, 12 l'un vers l'autre en les maintenant constamment aboutés.

Dans certains cas, les soudures 112, 122 à l'extrémité des tubes 11, 12 peuvent avoir été réalisées de manière trop forte (épaisseur de soudage trop importante et/ou surface de soudage (I x L) trop importante), ce qui peut ralentir de manière préjudiciable le procédé de raccordement, la durée de chauffage des soudures devant être augmentée.

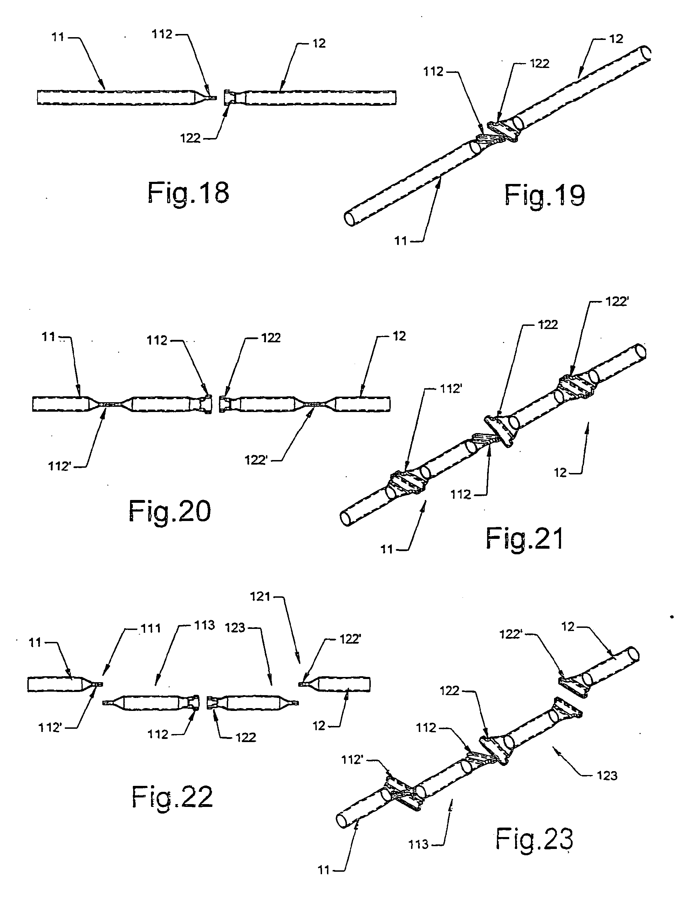

Pour pallier à ce problème, dans une variante de réalisation de l'invention, on peut, préalablement à la 1^{ère} étape précitée, réaliser les opérations additionnelles suivantes, qui vont être décrites en reference aux figures 18 à 23.

On commence par réaliser sur les deux tubes 11, 12, qui sont fermés par des soudures d'extrémité 112, 122 (figures 18 et 19), deux soudures HF secondaires 112', 122' (figures 20 et 21). Ces deux soudures 112', 122' sont réalisées de manière parfaitement contrôlée, et de manière moins forte que les soudures d'origine 112, 122, en sorte de réduire la contrainte de dessoudage ultérieure. Bien entendu, ces soudures secondaires 112', 122' doivent néanmoins être suffisantes pour assurer une fermeture étanche des tubes 11, 12.

Une fois les soudures secondaires 112', 122' réalisées, on découpe, par exemple au moyen d'une lame coupante ou d'un laser, les extrémités de chaque tube 11, 12 au niveau des soudures secondaires 112' et 122', et on retire les portions d'extrémités 113 et 123 des deux tubes 11, 12, comportant les soudures d'origine 112, 122.

Ensuite, on peut mettre en oeuvre les étapes précitées du procédé de l'invention qui ont été décrites précédemment (1^{ère} étape, 2^{ème} étape, ...) sur les tubes 11 et 12 fermés par des soudures d'extrémité 112', 122'.

Le cas échéant, préalablement à la réalisation des soudures secondaires 112', 122', il peut être nécessaire de clamper les tubes 11 et 12 dans le but d'évacuer du liquide contenu dans les extrémités des tubes 11 et 12.

## Revendications

1. Procédé de raccordement bout à bout de deux tubes (11), (12) en matière plastique dont les extrémités (111), (121) sont fermées par écrasement et soudure ou collage de la paroi du tube, **caractérisé en ce qu'**on aligne axialement et on aboute les deux extrémités (111), (121) fermées des tubes (11),(12), on chauffe à distance et sans contact les deux extrémités (111), (121) fermées et aboutées des tubes (11), (12) et on rapproche axialement les deux extrémités (111), (121) des tubes (11), (12), en sorte d'évaser et de souder ensemble bout à bout les extrémités (111), (121) des tubes (11), (12), tout en maintenant lesdites extrémités (111), (121) constamment aboutées.

2. Procédé selon la revendication 1, **caractérisé en ce que** pour chauffer à distance et sans contact les deux extrémités (111), (121) fermées et aboutées des tubes (11), (12), on utilise un faisceau laser (FL).

3. Procédé selon la revendication 2, **caractérisé en ce que** le faisceau laser (FL) touche simultanément les deux extrémités fermées et aboutées (111), (121) des tubes (11), (12).

4. Procédé selon la revendication 3, **caractérisé en ce que** le faisceau laser (FL) est orienté transversalement au plan d'écrasement (P) des deux extrémités (111), (121).

5. Procédé selon la revendication 4, **caractérisé en ce que** le faisceau laser (FL) effectue un balayage des extrémités (111), (121) fermées et aboutées des tubes (11), (12) suivant une direction (T) transversale à l'axe (A) des tubes (11), (12).

6. Procédé selon l'une des revendications 1 à 5, **caractérisé en ce que** pour chauffer à distance et sans contact les deux extrémités (111), (121) fermées et aboutées des tubes (11), (12), on utilise un flux d'air chaud.

7. Procédé selon l'une des revendications 1 à 6, **caractérisé en ce que** pour chauffer à distance et sans contact les deux extrémités (111), (121) fermées et aboutées des tubes (11), (12), on utilise un rayonnement micro-ondes.

8. Procédé selon l'une des revendications 1 à 7, **caractérisé en ce que** pour chauffer à distance et sans contact les deux extrémités (111), (121) fermées et aboutées des tubes (11), (12), on utilise un rayonnement infrarouge.

9. Procédé selon l'une des revendications 1 à 5, **caractérisé en ce que** pour chauffer à distance et sans contact les deux extrémités (111), (121) fermées et aboutées des tubes (11), (12), on utilise un faisceau laser (FL) et l'un au moins des moyens choisis parmi la liste suivante : flux d'air chaud, rayonnement micro-ondes, rayonnement infrarouge.

10. Procédé selon l'une des revendications 1 à 9, **caractérisé en ce qu'**on chauffe à distance et sans contact les deux extrémités (111), (121) fermées et aboutées des tubes (11), (12) à l'intérieur d'une enceinte (E).

11. Procédé selon l'une des revendications 1 à 10, **caractérisé en ce qu'**on détecte la température des extrémités (111), (121) des tubes (11), (12) et on commande le rapprochement des tubes (11), (12) en fonction de cette détection de température.

12. Procédé selon l'une des revendications 1 à 11, **caractérisé en ce que** les extrémités (111), (121) sont initialement fermées par soudure haute fréquence.

13. Procédé selon l'une des revendications 1 à 12, **caractérisé en ce que** postérieurement au raccordement des deux tubes (11), (12) on exerce une traction sur les tubes raccordés.

14. Procédé selon l'une des revendications 1 à 13, **caractérisé en ce que** les tubes (11), (12) sont en PVC.

15. Dispositif (1) de raccordement de tubes, permettant la mise en oeuvre du procédé visé à l'une des revendications 1 à 14, et comprenant, des moyens d'aboutement permettant un alignement axiale et un aboutement de deux tubes (11, 12), et des moyens de chauffage sans contact, **caractérisé en ce que** les moyens d'aboutement sont conçus pour déplacer automatiquement en translation l'une vers l'autre les deux extrémités aboutées des deux tubes (11,12) pendant la mise en oeuvre des moyens de chauffage (3, 3').

16. Dispositif selon la revendication 15, **caractérisé en ce qu'**il comprend des moyens optiques (2) de détection de température positionnés à proximité de la zone d'aboutement des tubes, et **en ce que** les moyens d'aboutement sont aptes à entraîner en translation les tubes en fonction du signal de détection de température délivré par lesdits moyens de détection (2).

17. Dispositif selon l'une des revendications 15 ou 16, **caractérisé en ce qu'**il comprend une enceinte de chauffage (E), et **en ce que** les moyens d'aboutement comportent des moyens (6, 7) d'entraînement en translation des tubes, qui sont à l'intérieur de l'enceinte.

18. Dispositif selon l'une des revendications 15 à 17, **caractérisé en ce que** les moyens d'aboutement comportent deux chariots alignés qui sont mobiles et guidés en translation, chaque chariot (6,7) étant équipé d'un moyen (61,71) permettant de fixer temporairement un tube (11, 12) sur le chariot, et des moyens d'actionnement permettant de rapprocher ou d'écarter les chariots (6,7).

19. Dispositif selon la revendication 18, **caractérisé en ce que** les moyens d'actionnement des chariots comportent une vis sans fin (13) et un moteur (M) bidirectionnel couplé à la vis et permettant d'entraîner la vis en rotation dans un sens ou dans l'autre.

20. Dispositif selon la revendication 18, **caractérisé en ce que** les moyens d'actionnement des chariots comportent pour chaque chariot (6,7) au moins un actionneur linéaire bidirectionnel, de type vérin (15).

21. Dispositif selon la revendication 18, **caractérisé en ce que** les moyens d'actionnement comportent des moyens élastiques exerçant constamment sur les chariots (6,7) des forces de rappel les repoussant l'un vers l'autre.

22. Dispositif selon l'une des revendications 15 à 21, **caractérisé en ce que** les moyens de chauffage sans contact comportent une source laser (3').

23. Dispositif selon l'une des revendications 15 à 22, **caractérisé en ce que** les moyens de chauffage sans contact comportent au moins une buse de soufflage d'air chaud (30).

24. Dispositif selon l'une des revendications 15 à 23, **caractérisé en ce que** les moyens de chauffage sans contact comportent au moins une source micro-ondes ou une source infrarouge.

25. Utilisation du procédé visé à l'une des revendications 1 à 14 ou du dispositif (1) visé à l'une des revendications 15 à 24, pour raccorder de manière stérile les tubes (11), (12) fermés de deux poches (P1, P5), dont l'une (P1) contient des plaquettes de sang.

## Claims

1. Method for connecting two plastic tubes (11), (12) end to end, the extremities (111), (121) of which are closed by compression and welding or gluing of the tube wall, **characterised in that** the two closed extremities (111), (121) of the tubes (11), (12) are axially aligned and placed in abutment, that the two closed and abutting extremities (111), (121) of the tubes (11), (12) are heated from a distance and without contact and that the two extremities (111), (121) of the tubes (11), (12) are axially brought together, so as to splay and weld together the extremities (111), (121) of the tubes (11), (12) end to end, while constantly maintaining said extremities (111), (121) in abutment.

2. Method according to claim 1, **characterised in that,** in order to heat at a distance and without contact the two closed and abutting extremities (111), (121) of the tubes (11), (12), a laser beam (FL) is used.

3. Method according to claim 2, **characterised in that** the laser beam (FL) simultaneously comes into contact with the two closed and abutting extremities (111), (121) of the tubes (11), (12).

4. Method according to claim 3, **characterised in that** the laser beam (FL) is orientated transversally to the compression plane (P) of the two extremities (111), (121).

5. Method according to claim 4, **characterised in that** the laser beam (FL) scans the closed and abutting extremities (111), (121) of the tubes (11), (12) according to a direction (T) transversal to the axis (A) of the tubes (11), (12).

6. Method according to one of claims 1 to 5, **characterised in that,** in order to heat at a distance and without contact the two closed and abutting extremities (111), (121) of the tubes (11), (12), a hot air stream is used.

7. Method according to one of claims 1 to 6, **characterised in that,** in order to heat at a distance and without contact the two closed and abutting extremities (111), (121) of the tubes (11), (12), microwave radiation is used.

8. Method according to one of claims 1 to 7, **characterised in that,** in order to heat at a distance and without contact the two closed and abutting extremities (111), (121) of the tubes (11), (12), infrared radiation is used.

9. Method according to one of claims 1 to 5, **characterised in that,** in order to heat at a distance and without contact the two closed and abutting extremities (111), (121) of the tubes (11), (12), a laser beam (FL) and at least one of the means from the following list are used: hot air stream, microwave radiation, infrared radiation.

10. Method according to one of claims 1 to 9, **characterised in that** the two closed and abutting extremities (111), (121) of the tubes (11), (12) are heated at a distance and without contact inside an enclosure (E).

11. Method according to one of claims 1 to 10, **characterised in that** the temperature of the extremities (111), (121) of the tubes (11), (12) is detected and the tubes (11), (12) are brought closer together in function of this temperature detection.

12. Method according to one of claims 1 to 11, **characterised in that** the extremities (111), (121) are initially closed by high-frequency welding.

13. Method according to one of claims 1 to 12, **characterised in that** subsequent to the connection of the two tubes (11), (12), traction is carried out on the connected tubes.

14. Method according to one of claims 1 to 13, **characterised in that** the tubes (11), (12) are made of PVC.

15. Device (1) for the connection of tubes that allows the implementation of the method referred to in one of claims 1 to 14 and including abutment means enabling the two tubes (11, 12) to be axially aligned and brought into abutment, and means of heating without contact, **characterised in that** the abutment means are designed to automatically displace the two extremities of the two tubes (11, 12) in abutment in a translation movement one towards the other during the implementation of the heating means (3, 3').

16. Device according to claim 15, **characterised in that** it includes optical temperature detection means (2) positioned close to the abutment zone of the tubes, and **in that** the abutment means are suitable for causing a translation movement of the tubes in function of the temperature detection signal issued by said detection means (2).

17. Device according to claim 15 or 16, **characterised in that** it includes a heating enclosure (E) and **in that** the abutment means include means (6, 7) for causing a translation movement of the tubes, which are inside the enclosure.

18. Device according to one of claims 15 to 17, **characterised in that** the abutment means include two aligned carriages, which are mobile and guided in translation movement, each carriage (6, 7) being equipped with a means (61, 71) for temporarily fixing a tube (11, 12) on the carriage, and with actuating means enabling the carriages (6, 7) to be brought closer together or further apart.

19. Device according to claim 18, **characterised in that** the actuating means of the carriages include an endless screw (13) and a two-way motor (M) coupled to the screw, allowing the screw to be rotated in one or the other sense.

20. Device according to claim 18, **characterised in that** the actuating means of the carriages include for each carriage (6, 7) at least one linear two-way actuator of the elevating screw type (15).

21. Device according to claim 18, **characterised in that** the actuating means include elastic means constantly exercising spring forces on the carriages (6, 7), pushing them towards one other.

22. Device according to one of claims 15 to 21, **characterised in that** the heating means without contact include a laser source (3').

23. Device according to one of claims 15 to 22, **characterised in that** the heating means without contact include at least one hot air blowing nozzle (30).

24. Device according to one of claims 15 to 23, **characterised in that** the heating means without contact include at least a microwave source or an infrared source.

25. Use of the method referred to in one of claims 1 to 14 or of the device (1) referred to in one of claims 15 to 24, for connecting in a sterile manner the closed tubes (11), (12) of two bags (P1, P5), one of which (P1) contains blood platelets.

## Patentansprüche

1. **Verfahren zum Verbinden** zweier Kunststoffrohre (11),(12) auf Stoß, deren Enden (111),(121) durch Abquetschen und Verschweißen bzw. Verkleben der Rohrwandung verschlossen sind, **dadurch gekennzeichnet, dass** die beiden verschlossenen Enden (111),(121) der Rohre (11),(12) axial ausgerichtet und aneinandergesetzt werden, dass die beiden verschlossenen und aneinandergesetzten Enden (111),(121) der Rohre (11),(12) im Abstand und berührungslos erhitzt werden und die beiden Enden (111),(121) der Rohre (11),(12) axial zusammengedrückt werden, so dass die Enden (111),(121) der Rohre (11),(12) erweitert und auf Stoß zusammengeschweißt werden, wobei die Enden (111),(121) stets aneinandergesetzt gehalten werden.

2. Verfahren nach Anspruch 1, **dadurch gekennzeichnet, dass** zum berührungslosen Erhitzen im Abstand von den beiden verschlossenen und aneinandergesetzten Enden (111),(121) der Rohre (11),(12) ein Laserstrahl (FL) verwendet wird.

3. Verfahren nach Anspruch 2, **dadurch gekennzeichnet, dass** die beiden verschlossenen und aneinandergesetzten Enden (111),(121) der Rohre (11),(12) gleichzeitig vom Laserstrahl (FL) berührt werden.

4. Verfahren nach Anspruch 3, **dadurch gekennzeichnet, dass** der Laserstrahl (FL) quer zur Quetschebene (P) der beiden Enden (111),(121) gerichtet ist.

5. Verfahren nach Anspruch 4, **dadurch gekennzeichnet, dass** mit dem Laserstrahl (FL) ein Abtasten der verschlossenen und aneinandergesetzten Enden (111),(121) der Rohre (11),(12) in einer Richtung (T) quer zur Achse (A) der Rohre (11),(12) erfolgt.

6. Verfahren nach einem der Ansprüche 1 bis 5, **dadurch gekennzeichnet, dass** zum berührungslosen Erhitzen im Abstand von den beiden verschlossenen und aneinandergesetzten Enden (111),(121) der Rohre (11),(12) ein Heißluftstrom verwendet wird.

7. Verfahren nach einem der Ansprüche 1 bis 6, **dadurch gekennzeichnet, dass** zum berührungslosen Erhitzen im Abstand von den beiden verschlossenen und aneinandergesetzten Enden (111),(121) der Rohre (11),(12) eine Mikrowellenstrahlung verwendet wird.

8. Verfahren nach einem der Ansprüche 1 bis 7, **dadurch gekennzeichnet, dass** zum berührungslosen Erhitzen im Abstand von den beiden verschlossenen und aneinandergesetzten Enden (111),(121) der Rohre (11),(12) eine Infrarotstrahlung verwendet wird.

9. Verfahren nach einem der Ansprüche 1 bis 5, **dadurch gekennzeichnet, dass** zum berührungslosen Erhitzen im Abstand von den beiden verschlossenen und aneinandergesetzten Enden (111),(121) der Rohre (11),(12) ein Laserstrahl (FL) und zumindest eines der Mittel aus nachfolgender Aufzählung verwendet werden: Heißluftstrom, Mikrowellenstrahlung, Infrarotstrahlung.

10. Verfahren nach einem der Ansprüche 1 bis 9, **dadurch gekennzeichnet, dass** die beiden verschlossenen und aneinandergesetzten Enden (111),(121) der Rohre (11),(12) im Inneren eines Gehäuses (E) berührungslos und mit Abstand erhitzt werden.

11. Verfahren nach einem der Ansprüche 1 bis 10, **dadurch gekennzeichnet, dass** die Temperatur der Enden (111),(121) der Rohre (11),(12) erfasst wird und das Zusammendrücken der Rohre (11),(12) in Abhängigkeit von dieser Temperaturerfassung gesteuert wird.

12. Verfahren nach einem der Ansprüche 1 bis 11, **dadurch gekennzeichnet, dass** die Enden (111),(121) zunächst durch Hochfrequenzschweißen verschlossen werden.

13. Verfahren nach einem der Ansprüche 1 bis 12, **dadurch gekennzeichnet, dass** nach dem Verbinden der beiden Rohre (11),(12) eine Zugkraft auf die verbundenen Rohre ausgeübt wird.

14. Verfahren nach einem der Ansprüche 1 bis 13, **dadurch gekennzeichnet, dass** die Rohre (11),(12) aus PVC sind.

15. **Vorrichtung** (1) zum Verbinden von Rohren, mit welcher das Verfahren nach einem der Ansprüche 1 bis 14 durchführbar ist, die Vorrichtung enthaltend Mittel zum Aneinandersetzen, die ein axiales Ausrichten und ein Aneinandersetzen zweier Rohre (11),(12) gestatten, sowie Mittel zum berührungslosen Erhitzen, **dadurch gekennzeichnet, dass** die Mittel zum Aneinandersetzen ausgelegt oder ausgebildet sind, die beiden aneinandergesetzten Enden der beiden Rohre (11),(12) während der Anwendung der Heizmittel (3,3') automatisch zueinander translatorisch zu verschieben.

16. Vorrichtung nach Anspruch 15, **dadurch gekennzeichnet, dass** sie optische Temperaturerfassungsmittel (2) aufweist, die in der Nähe des Aneinandersetzbereichs der Rohre positioniert sind, und dass die Ansetzmittel die Rohre in Abhängigkeit von dem von den Erfassungsmitteln (2) ausgegebenen Temperaturerfassungssignal translatorisch mitnehmen können.

17. Vorrichtung nach einem der Ansprüche 15 oder 16, **dadurch gekennzeichnet, dass** sie ein Heizgehäuse (E) aufweist und dass die Ansetzmittel Mittel (6,7) zum translatorischen Mitnehmen der Rohre aufweisen, die sich im Inneren des Gehäuses befinden.

18. Vorrichtung nach einem der Ansprüche 15 bis 17, **dadurch gekennzeichnet, dass** die Ansetzmittel zwei fluchtende Schlitten aufweisen, die beweglich und verschiebbar geführt sind, wobei jeder Schlitten (6,7) mit einem Mittel (61,71) ausgestattet ist, mit welchem ein Rohr (11),(12) vorübergehend an dem Schlitten fixiert werden kann, sowie Betätigungsmittel, mit denen die Schlitten (6,7) einander angenähert bzw. voneinander entfernt werden können.

19. Vorrichtung nach Anspruch 18, **dadurch gekennzeichnet, dass** die Mittel zum Betätigen der Schlitten eine Schnecke (13) und einen mit der Schnecke gekoppelten bidirektionalen Motor (M) aufweisen, mit dem die Schnecke in der einen oder anderen Richtung drehend angetrieben werden kann.

20. Vorrichtung nach Anspruch 18, **dadurch gekennzeichnet, dass** die Mittel zum Betätigen der Schlitten für jeden Schlitten (6,7) zumindest ein bidirektionales Linearbetätigungsglied vom Typ Kraftzylinder (15) aufweisen.

21. Vorrichtung nach Anspruch 18, **dadurch gekennzeichnet, dass** die Betätigungsmittel Federmittel aufweisen, die stets Rückstellkräfte auf die Schlitten (6,7) ausüben, wodurch sie zueinander verschoben werden.

22. Vorrichtung nach einem der Ansprüche 15 bis 21, **dadurch gekennzeichnet, dass** die Mittel zum berührungslosen Erhitzen eine Laserquelle (3') aufweisen.

23. Vorrichtung nach einem der Ansprüche 15 bis 22, **dadurch gekennzeichnet, dass** die Mittel zum berührungslosen Erhitzen zumindest eine Heißluftblasdüse (30) aufweise.

24. Vorrichtung nach einem der Ansprüche 15 bis 23, **dadurch gekennzeichnet, dass** die Mittel zum berührungslosen Erhitzen zumindest eine Mikrowellenquelle bzw. Infrarotquelle aufweisen.

25. **Anwendung** des Verfahrens nach einem der Ansprüche 1 bis 14 oder der Vorrichtung (1) nach einem der Ansprüche 15 bis 24 zum sterilen Verbinden der verschlossenen Rohre (11),(12) zweier Beutel (P1,P5), von denen der eine (P1) Blutplättchen enthält.
